# EUROPEAN PATENT APPLICATION

(11) **EP 4 851 273 A1**
(43) Date of publication of application: **09.12.2026**
(21) Application number: 25180544.6
(22) Date of filing: 03.06.2025
(51) Int. Cl.: C12M 3/00, C12M 1/00

(54) **BIOPROCESSING SYSTEM FOR PERFORMING A CONNECTED BIOPROCESS OF A PROCESS LIQUID**

(71) Applicant: Sartorius Stedim Biotech GmbH, 37079 Göttingen (DE)
(72) Inventor: Stein, Dominik, 37079 Göttingen (DE)
(74) Representative: Gottschald Patentanwälte Partnerschaft mbB

(57) **Abstract**

The invention relates to a bioprocessing system for performing a connected bioprocess (2) of a process liquid, wherein the bioprocessing system (1) comprises a group of modules (3), wherein the group of modules (3) comprises a start module (4) and/or an end module (5) for performing support functions during the connected bioprocess (2), wherein the group of modules (3) comprises a flexible number of operation modules each being functionally arranged behind the start module (4) and/or in front of the end module (5), wherein the bioprocessing system (1) is configured to perform a flexible number of unit operations by the flexible number of operation modules, wherein the start module (4) and/or the end module (5) is or are configured to perform the support functions for each operation module (6), wherein each module of the group of modules (3) comprises a group of predefined physical interfaces for fluidically and/or electrically interconnecting the modules of the group of modules (3) with each other, wherein the bioprocessing system (1) has a footprint (8) that is defined by the interaction of the physical interfaces (7) of the group of predefined physical interfaces.

## Description

The present invention relates to a bioprocessing system for performing a connected bioprocess of a process liquid according to claim 1, to a method for setting up a bioprocessing system according to claim 14, and to a process unit for controlling a bioprocess system according to claim 15.

A known bioprocessing system for performing a connected bioprocess of a process liquid according to WO 2024/072963 A1 is related to a bioprocessing system which comprises multiple modular assemblies as modules, wherein each module may be configured to perform a unit operation of a bioprocess, such as a chromatography step, a virus inactivation step, a virus filtration step, and an ultrafiltration step. These modules can either be coupled directly with each other or with other processing steps in between. The modules may, for example, be coupled by means of tubing.

Another bioprocessing system for performing a connected bioprocess of a process liquid is known from WO 2020/065040 A1, which comprises a processing control element and a processing interface with a fluid processing device. The processing interface is connected to the processing control element, e.g., by means of tubing.

Although the bioprocessing systems of the prior art all comprise a safe and tight connection between the different modules, the overall bioprocessing system requires a large footprint for its installation. As the connection by tubing requires additional space between each and every module, a compact installation is nearly impossible. Furthermore, although the tubing of the known bioprocessing systems allows flexibly arranging the different modules, it is not possible to provide a predetermined orientation of the modules in relation to each other, and thus a predetermined spatial configuration of the system. In addition, as connecting the different modules with each other by means of tubing requires hands-on work, the installation of the known bioprocessing systems is time-consuming and error-prone.

It is therefore an object of the invention to provide a bioprocessing system with a reduced footprint that allows a simple and failure-proof installation.

The above-noted object is solved by the features of claim 1.

The main realization of the present invention is that by replacing the connecting tubing and/or wiring between the modules with physical interfaces for fluidically and/or electrically interconnecting the modules with each other, gaps resulting from tubing and/or wiring may no longer be required, which may ultimately result in a drastically reduced footprint of the bioprocessing system by a compact arrangement of the modules to each other. As the orientation of the physical interfaces on each module also determines the orientation of adjacent modules after their connection, it becomes possible to predetermine the spatial configuration of the whole bioprocessing system and thus its footprint in spatial extension. Furthermore, the physical interfaces allow a simple connection of adjacent modules with each other and thereby a simple installation of the whole bioprocessing system, for example, by simply pushing the modules together.

In addition, due to the simple connection of two or more modules by means of the physical interfaces, it becomes possible to easily add modules to or remove modules from a given bioprocessing system, thereby allowing flexibly adjusting the size of the bioprocessing system according to the needs. This further allows simply adding analytic modules, which may simplify or even enable implementation of process analytical technology (PAT) in existing or planned connected bioprocesses.

Proposed is a bioprocessing system for performing a connected bioprocess of a process liquid, wherein the bioprocessing system comprises a group of modules, wherein the group of modules comprises a start module and/or an end module for performing support functions during the connected bioprocess, wherein the group of modules comprises a flexible number of operation modules each being functionally arranged behind the start module and/or in front of the end module, wherein the bioprocessing system is configured to perform a flexible number of unit operations by the flexible number of operation modules, wherein the start module and/or the end module is or are configured to perform the support functions for each operation module, wherein each module of the group of modules comprises a group of predefined physical interfaces for fluidically and/or electrically interconnecting the modules of the group of modules with each other, wherein the bioprocessing system has a footprint that is defined by the interaction of the physical interfaces of the group of predefined physical interfaces.

The bioprocessing system may be configured to perform an upstream process comprising at least one upstream processing step and/or at least one downstream process comprising at least one downstream processing step. An upstream process step is any process step which results in the formation of the target product, whereas a downstream process step is any process step which results in a purification of a target product.

In case of an upstream process, the term process liquid refers to any liquid which serves as a basis for the formation of a target product in the course of the connected bioprocess such as a cultivation medium. In case of a downstream process, the term process liquid refers to any liquid which comprises the target product.

The term "target product" refers to any desired product and may be a cell, a protein, in particular an antibody, and/or, a nucleic acid, such as a deoxyribonucleic acid or a ribonucleic acid. The target product may be a virus, such as an adeno-associated virus.

The term "unit operation" refers to any process step that results in a permanent change of a physical and/or chemical property of the process liquid and/or in a permanent change of the composition of the process liquid. The unit operation may result in the formation of the target product and/or an enrichment of the target product, and/or in an increase in purity of the target product. The unit operation may, for example, comprise a chromatography step, a filtration step, a virus inactivation step, or a centrifugation step.

A support function is any function which is not a unit operation.

The support functions performed by the start module and/or the end module may comprise one or more controlling support functions, which refer to a function required for the functioning and/or the controlling of a module of the group of modules, in particular an operation module. A controlling support function may comprise the provision of an electric signal, the provision of a pneumatic fluid and/or the provision of electricity.

The support functions may also comprise one or more process-liquid related support functions, which refer to a function, which involves a direct or indirect interaction with the process liquid. A process liquid-related support function may comprise a detection of the target product and/or an impurity comprised by the process liquid, a transport of the process liquid, for example by means of a pump, a sampling of the process liquid, a cooling of the process liquid, a heating of the process liquid and/or the removal of gas from the process liquid (venting).

Furthermore, the support functions may comprise one or more assisting support functions, which refer to a function which involves a direct or indirect interaction with a substance required for performing a unit operation. An assisting support function may comprise the transport of a fluid required for performing a unit operation, such as the transportation of a buffer, and/or of the transport of a solid material, such as a particular filter aid.

The term "footprint" as used herein is understood as a geometrical and/or spatial footprint.

In preferred embodiments, according to claim 2, the group of modules comprises a support module which allows assigning additional support functions to modules which are functionally arranged behind the start module and/or in front of the end module.

According to embodiments of claim 3, the group of predefined physical interfaces comprises a group of electric connectors. Alternatively, or in addition, the group of predefined physical interfaces comprises a group of fluidic connectors. If the group of electric connectors and/or the group of fluidic connectors are detachable plug-socket connectors, two or more modules can be connected with each other in a particularly simple and failure-proof manner. Furthermore, such connectors may further facilitate the connection of multiple modules by means of pushing them together. If all electric connectors and/or fluid connectors are of the same type, a particularly cost-effective system can be provided by means of standardization. Such embodiments may further facilitate plug-and-play connection of multiple modules, wherein one, in particular all, of the modules of the group of modules are ready-to-operate when being connected, with little or no manual configuration or setup required by a user.

**In** a preferred embodiment according to claim 4, the number of operation modules comprises a coupling operation module and/or the number of support modules comprises a coupling support module. These coupling modules are configured to receive the electric signal and/or the fluid from a transmitting module and to transmit the electric signal and/or the fluid to a receiving module which may allow for a transfer of a fluid and/or an electric signal from one module to another module.

Claim 5 specifies the modules from which the electric signal and/or fluid is received and to which the electric signal and/or fluid is transmitted by the coupling module.

Claim 6 specifies the electric signal as a control signal which allows controlling a module of the group of modules, thereby enabling the execution of necessary functions of one or more unit operations, e.g. the control of a pump. Alternatively, or in addition, the electric signal may be a detector signal which allows transmitting process parameters, such as a parameter representing the concentration of a target product or an impurity, from one module to another one.

According to preferred embodiments, a module of the group of modules comprises a pump and/or a chromatography medium and/or a filter medium and/or a detector for generating the detector signal (claim 7).

**In** embodiments according to claim 8, one unit operation is assigned to one operation module. Such embodiments allow setting up a bioprocessing system with a very low number of modules to be connected which may facilitate a quick and failure-proof installation of the bioprocessing system.

According to preferred embodiments of claim 9, each module of the group of modules comprises an arrangement surface for assigning the relative position of the modules to each other in which the group of predefined physical interfaces are arranged. Such embodiments may simplify the connection process of multiple modules and may allow predefining the footprint of the bioprocessing system in its spatial extension. In particular in combination with detachable plug-socket electric and/or fluidic connectors such embodiments enable a simple, fast and failure-proof connection of two or more modules.

In embodiments according to claim 10, a module of the group of modules comprises a housing for housing at least one sub-module, which is configured to perform an operation, in particular a unit operation. Embodiments in which the sub-module can be inserted, in particular be slid, into the housing, may allow preparing an operation of the connected bioprocess by equipping a sub-module with respective components required for this operation way before starting the connected bioprocess. Furthermore, such embodiments allow a pre-sterilization of the components together with the sub-module, which may protect the modules, in particular sensitive parts of the modules such as electronics, from the harsh conditions of a sterilization process. After the sub-module has been equipped with components and possibly sterilized together with the components, the sub-module can be inserted into a module of the module group just before starting the actual connected bioprocess, which may result in further time savings. Arranging the physical interfaces of the group of predefined physical interfaces on the housing (claim 11) allows providing interconnectable modules in which sensitive electronics and/or fluidics are safely housed in the interior, shielded from external influences.

Claim 12 concerns a preferred configuration of the bioprocessing system in which the operation modules are spatially arranged between the start module and the end module.

According to preferred embodiments of claim 13, all modules have a common, in particular planar, surface. If the common surface is formed by a top surface of the housing of at least two modules an additional workspace and/or storage area for preparation and/or storage of additional components can be provided.

Such embodiments may result in an in particularly compact bioprocessing system with a very low footprint, as components, which normally need to be placed next to the bioprocessing system, such as a personal computer and/or a display, can be placed on top of the bioprocessing system.

Another teaching according to claim 14, which is of equal importance, relates to a method for setting up a proposed bioprocessing system, wherein the method comprises configuring the bioprocessing system for performing a first connected bioprocess by interconnecting a start module and at least two operation modules of the number of operation modules via the physical interfaces of the group of predefined physical interfaces and reconfiguring the bioprocessing system for performing a second connected bioprocess by adding or removing one of the operation modules.

All explanations given with regard to the bioprocessing system are fully applicable.

Another teaching according to claim 15, which is of equal importance, relates to a process unit for controlling the proposed bioprocess system.

All explanations given with regard to the bioprocessing system and the method are fully applicable.

In the following, embodiments of the invention are explained with respect to the drawing. The drawing shows in
- Fig. 1: a schematic illustration of a first embodiment of the proposed bioprocessing system comprising a start module, two operation modules and one end module in a first scale,
- Fig. 2: a) a schematic illustration of a second embodiment of the proposed bioprocessing system as the bioprocessing system of Fig. 1 in a second scale and
- Fig. 3: a) a schematic representation of a third embodiment of the proposed bioprocessing system comprising a start module, a support module, an operation module and an end module in a top view with shown internal components and b) a schematic illustration of the footprint of the proposed bioprocessing system of Fig. 3a).

Proposed is a bioprocessing system 1 for performing a connected bioprocess 2 of a process liquid. Preferably, the connected bioprocess 2 is a closed connected bioprocess 2. The term "closed connected bioprocess" refers to a connected bioprocess 2 in which all components of the bioprocess are connected to each other in such a way that the target product is never exposed to the external environment.

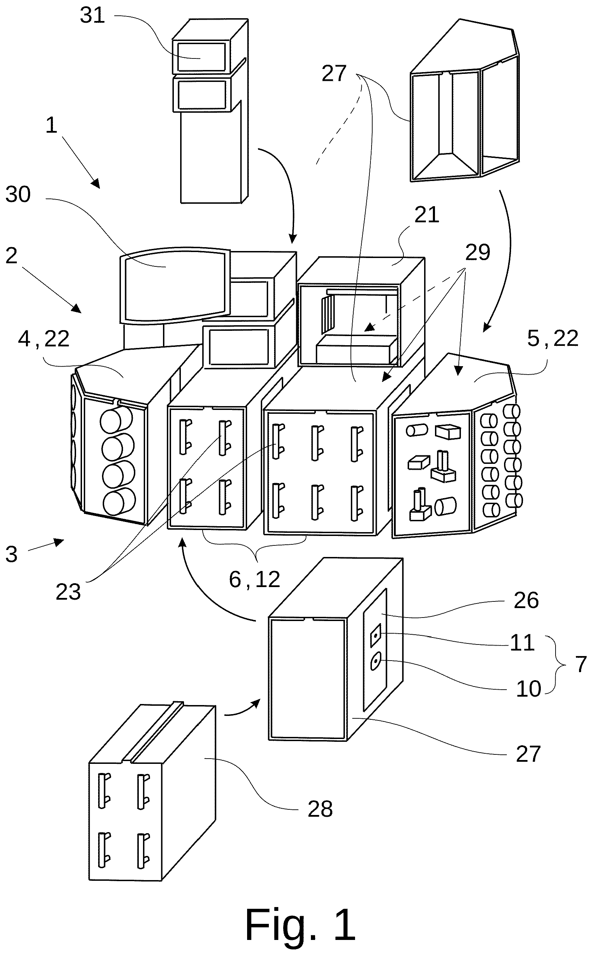

As shown in Figures 1 to 3, the bioprocessing system 1 comprises a group of modules 3, wherein a module is any distinct and in particular self-contained unit or device that is designed to carry out a defined function of the bioprocessing system 1. A module of the group of modules 3 may have any shape. For example, a module of the group of modules 3 may be triangular, quadratic, trapezoidal, hexagonal or octagonal in a top view.

The group of modules 3 comprises a start module 4 and/or an end module 5 for performing support functions during the connected bioprocess 2. Preferably, and as shown in Figures 1 to 3, the bioprocessing system 1 comprises a start module 4 (leftmost module) and an end module 5 (rightmost module).

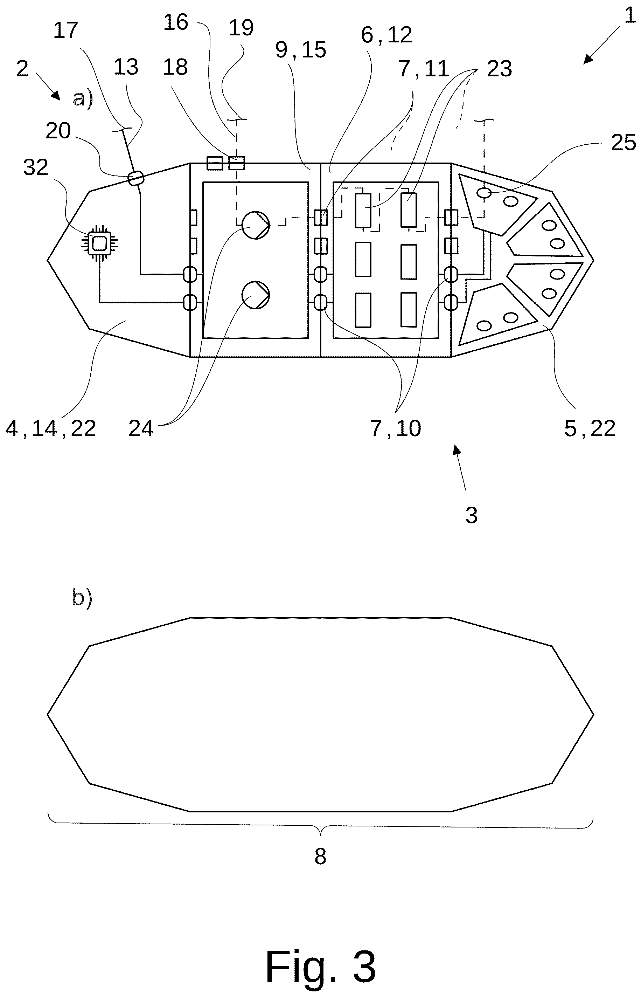

As shown in Figures 1 to 3, the group of modules 3 further comprises a flexible number of operation modules, each being functionally arranged behind the start module 4 and/or in front of the end module 5. For example, the bioprocessing system 1 may comprise one operation module 6 (Fig. 3, second module from the right) or two operation modules 6 (Fig. 1, modules between the start module 4 and the end module 5). However, three, four or more operation modules 6 are conceivable. Here and preferably, the operation modules 6 are arranged in a chain behind the start module 4 and/or in front of the end module 5.

The bioprocessing system 1 is further configured to perform a flexible number of unit operations by the flexible number of operation modules, for example by adding or removing operation modules 6.

A module of the group of modules 3 may comprise electric and/or mechanic and/or pneumatic operational components which enable or facilitate the functioning of the module. Such operational components may include a module-internal valve and/or a module-internal fluid network for module-internal fluid orchestration and/or a module-internal electric wiring. In some embodiments, the operational components may be exchangeable, for example, to meet good manufacturing process (GMP) criteria. The operational components may be single-use or multi-use components.

The start module 4 and/or the end module 5 is or are configured to perform the support functions for each operation module 6. Any or all of the support functions mentioned in the introduction may be performed by the start module 4 and/or the end module 5.

As shown in Fig. 3a) each module of the group of modules 3 furthermore comprises a group of predefined physical interfaces for fluidically and/or electrically interconnecting the modules of the group of modules 3 with each other. In a mounted state of the bioprocessing system 1, all modules of the group of modules 3 may therefore be interconnected with each other by means of predefined physical interfaces 7 of the group of predefined physical interfaces.

The footprint 8 of the bioprocessing system 1 is defined by the interaction of the physical interfaces 7 of the group of predefined physical interfaces. The term "interaction" means the arrangement of the physical interfaces 7 on the modules and/or the orientation of the physical interfaces 7 of different modules to each other. The term "footprint" 8 as used herein is defined as the shape and area of floor space required to install and operate the bioprocessing system 1, which is represented by the circumferential extension of the bioprocessing system 1 in a top view. Preferably, the physical interfaces 7 of the group of predefined physical interfaces of each module of the group of modules 3 are arranged in mating connector orientation relative to the physical interfaces 7 of the other modules of the group of modules 3, which facilitates the modules of the group of modules 3 to be connected by pushing them together.

As the modules of the group of modules 3 can be interconnected with each other by means of the physical interfaces 7 of the group of physical interfaces 7 only, and may thereby be free of further fluidic connection elements, such as tubing, and/or, electric connection elements, such as wiring, a bioprocessing system 1 with very small footprint 8 can be provided.

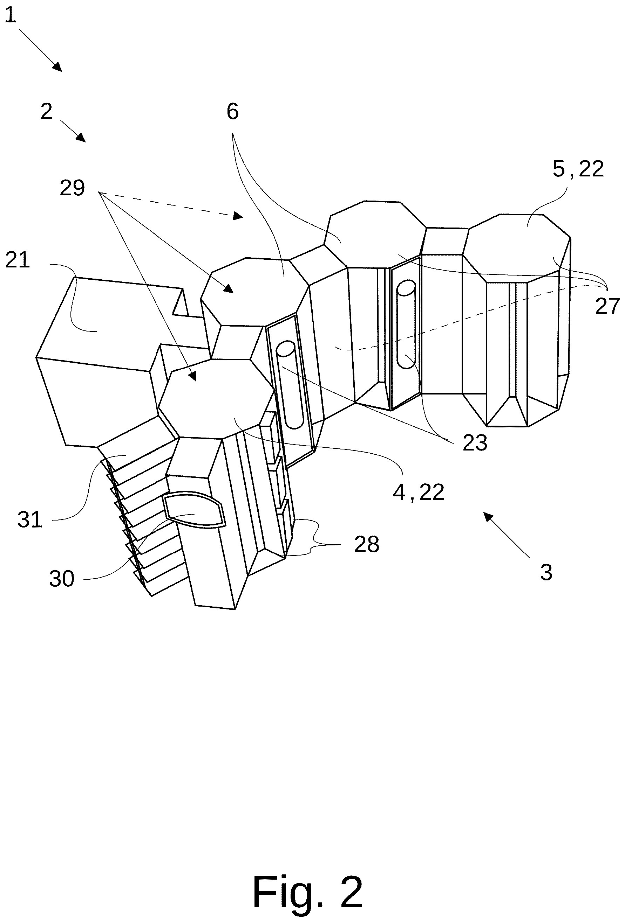

Furthermore, the modules of the group of modules 3 of the proposed bioprocessing system 1 may be provided in at least two different scales, in particular in a laboratory scale and/or a pilot scale or process scale. This is shown in Figures 1 and 2 where Fig. 1 shows a first embodiment of the bioprocessing system 1 comprising a group of modules 3 with a start module 4 (leftmost module), an end module 5 (rightmost module) and two operation modules 6 (modules between the start module 4 and the end module 5) in a first scale and where Fig. 2 shows a second embodiment of the bioprocessing system 1 comprising a group of modules 3 in a larger scale of the modules of the group of modules 3 of the first embodiment (Fig.2).

Providing the modules of the group of modules 3 in the at least two different scales allows to simply and failure-proof scale a given connected bioprocess 2 from one scale to another scale, in particular from lab-scale to pilot or process scale. This allows optimizing a desired connected bioprocess 2 in a small scale and to easily setting up the desired connected bioprocess 2 in a large scale by means of optimized and scaled process parameters obtained in the small scale.

According to one embodiment it is proposed that the group of modules 3 comprises a flexible number of support modules for performing support functions during the connected bioprocess 2, which are functionally arranged behind the start module 4 and/or in front of the end module 5. The support functions may, for example, comprise a controlling support function, and/or a process liquid-related support function, and/or an assisting support function. The support modules 9 may perform additional support functions not performed by the start module 4 and the end module 5.

For example and as shown in Fig. 3a), the bioprocessing system 1 may comprise one support module 9. However, other embodiments may comprise two, three, four or more support modules 9.

As shown in Fig. 3, the group of predefined physical interfaces may comprise a group of electric connectors, in particular detachable, electrical plug-socket connectors, for electrically interconnecting the modules of the group of modules 3 with each other. Preferably, all electric connectors 10 of the group of electric connectors are of the same type.

Alternatively, or in addition, the group of predefined physical interfaces may comprise a group of fluidic connectors, in particular detachable, fluidic plug-socket connectors, for fluidically interconnecting the modules of the group of modules 3 with each other. Preferably, all fluidic connectors 11 of the group of fluid connectors are of the same type.

It is in particularly preferred, that all electric connectors 10 of the group of connectors are detachable, electrical plug-socket connectors, that all fluidic connectors 11 of the group of fluidic connectors are detachable, fluidic plug-socket connectors, and that all electric connectors 10 of the group of connectors and all fluidic connectors 11 of the group of fluid connectors are of the same type. Such an embodiment facilitates a simple connection of all modules of the group of modules 3.

Detachable connectors allow to easily adapt the bioprocessing system 1 to the requirements of a desired connected bioprocess 2 as modules can simply be added or removed by connecting new modules or disconnecting modules that are already part of a given connected bioprocess 2. For example, this allows easily adding purification steps to or removing purification steps from a connected bioprocess 2. The connection or disconnection of modules may be achieved manually or automatically.

In this regard, it is made clear that an electric connection and/or a fluid connection allows electricity or a fluid to be transmitted from one module of the group of modules 3 to another one. Thus, a permanent fluid flow or a permanent transmission of electricity between interconnected modules is not required.

As can be derived from Figures 3a) and 3b) the location and orientation of the electric connectors 10 of the group of electric connectors of each module and the fluid connectors of the group of fluidic connectors of each module between adjacent and connected modules (Fig. 3a)) define the footprint 8 of the bioprocessing system 1 (Fig. 3b)).

In other embodiments, group of predefined physical interfaces comprises a group of tube elements for fluidically interconnecting the modules of the group of modules 3 with each other. Here and preferably, a module of the group of modules 3 comprises a welding device (not shown) for welding the tube elements between at least two modules of the group of modules 3 in order to fluidically interconnect the at least two modules of the group of modules 3 with each other.

As shown in Fig. 1, the number of operation modules may comprise a coupling operation module 12 which is an operation module 6 configured to receive an electric signal 13 and/or a fluid from a transmitting module of the group of modules 3 and to transmit the electric signal 13 and/or the fluid to a receiving module of the group of modules 3 via the physical interfaces 7 of the group of predefined physical interfaces of the transmitting module, the coupling operation module 12 and the receiving module. Alternatively, or in addition, the number of support modules may comprise a coupling support module 9 which is a support module 9 configured to receive an electric signal 13 and/or a fluid from a transmitting module of the group of modules 3 and to transmit the electric signal 13 and/or the fluid to a receiving module of the group of modules 3 via the physical interfaces 7 of the group of predefined physical interfaces of the transmitting module, the coupling support module 9 and the receiving module. The fluid may be the process liquid and/or a fluid required for performing a unit operation.

The electric signal 13 and/or the fluid may be transmitted to a receiving module, which is located in downstream direction. The term "downstream direction" as used herein is the direction of a signal and/or fluid in spatial direction to the last module of the group of modules 3. Alternatively, the electric signal 13 and/or the fluid may be transmitted to a receiving module which is located in upstream direction. The term "upstream direction" as used herein is the direction of a signal and/or fluid in spatial direction to the first module of the group of modules 3.

In some embodiments, the electric signal 13 is received from and in particular originates from the start module 4 and/or the end module 5 and/or an operation module 6 and/or a support module 9 as an electric source module 14. Alternatively, or in addition, the fluid is received from the start module 4 and/or the end module 5 and/or an operation module 6 and/or a support module 9 as a fluid source module 15.

Generally, the start module 4 and/or the end module 5 may control a process step or unit operation performed by one of the operation modules 6 or several or all of the operation modules 6. The controlling may comprise sending actuation signals to actuate actuators like pumps or valves of the operation module 6 or operation modules 6 and/or sending process parameters like a temperature or a flow rate to the operation module 6 or operation modules 6 which the operation module 6 or operation modules 6 then implement, in particular as a target in the process step or unit operation. To control an operation module 6 not directly connected to the start module 4 or the end module 5, the start module 4 or end module 5 may send the electric signal 13 through the coupling operation module 12 or coupling support module 9. This shows how a chain of operation modules 6 can be controlled by the start module 4 and/or the end module 5 and underlines how a flexible number of unit operations can be performed.

It is preferred, that the fluid received from the fluid source module 15, in particular from an operation module 6 as fluid source module 15, is introduced into the fluid source module 15 from an external feedstream 16. Preferably, the external feedstream 16 is the process liquid derived from an upstream process as shown in Fig. 3a). It is conceivable, that the electric signal 13 received from the electric source module 14 is introduced into the electric source module 14 from an external electric signal source 17 (Fig. 3). Independent of its source, the process liquid is preferably passed from one operation module 6 to another operation module 6, in particular directly. The process liquid may be passed to several operation modules 6 in a chain or even to all operation modules 6, without leaving the bioprocessing system 1.

For receiving the fluid, the fluid source module 15 may comprise a group of fluid source interfaces with at least one fluid source interface 18 for fluidically connecting the fluid source module 15 with a fluid source 19 from which the external feedstream 16 is derived. Accordingly, for receiving the electric signal 13, the electric source module 14 may comprise a group of electric source interfaces with at least one electric source interface 20 for electrically connecting the electric source module 14 with the electric signal source 17, e.g. an electric signal 13 provided by a liquid handling station 21. However, in some embodiments it may be the preferred, that a fluid source interface 18 of the group of fluid source interfaces is configured to allow an exchange of fluid between the fluid source module 15 and the fluid source 19 and/or that an electric source interface 20 of the group of electric source interfaces is configured to allow an exchange of an electric signal 13 between the electric source module 14 and the electric signal source 17. Such embodiments may enable a communication in both directions between the fluid source module 15 and the fluid source 19 and/or the electric source module 14 and the electric signal source 17, such as a further electric device, for example the liquid handling station 21.

In this regard, it is preferred that the group of electric source interfaces comprises a group of external electric connectors (not shown), in particular detachable, electrical plug-socket connectors, and/or, that the group of fluid source interfaces comprises a group of external fluidic connectors, in particular detachable, fluidic plug-socket connectors. It is further preferred, that the external electric connectors of the group of external electric connectors are of the same type as the electric connectors 10 of the group of electric connectors, and/or, that the external fluidic connectors of the group of external fluidic connectors are of the same type as the fluidic connectors 11 of the group of fluidic connectors. Such embodiments allow an easy connection of the feedstream to the fluid source module 15 and/or the electric source module 14 to an electric signal source 17.

According to one embodiment, it is proposed that the electric signal 13, in particular when stemming from the start module 4 or the end module 5, is a control signal for controlling a module of the group of modules 3, preferably, for controlling a unit operation of an operation module 6 of the group of operation modules 6. Alternatively, or in addition, in particular when sent to and processed by the start module 4 or the end module 5, the electric signal 13 may be a detector signal representing a bioprocess parameter of the process liquid. A bioprocess parameter of the process liquid is any parameter measurable by a detector that influences or characterizes the conditions, the performance or the outcome of the connected bioprocess 2, such as the pH and/or the temperature and/or the conductivity of the process liquid or the concentration of a substance comprised by the process liquid, as for example the target product and/or the impurity.

The start module 4 and/or the end module 5 and/or a support module 9 of the number of support modules may be a control module 22, which is configured to perform the controlling support function of the support functions.

It is preferred, that the start module 4 and/or the end module 5 and/or a support module 9 of the number of support modules, in particular the control module 22, is configured to automatically recognize a newly connected module of the group of modules 3, and/or to automatically supply a newly connected module of the group of modules 3, with electricity, and/or to automatically check the condition of a newly connected module and/or to automatically install a driver to a newly connected module. Such a plug and play approach further facilitates a simple and failure-proof installation of the bioprocessing system 1.

According to one embodiment it is proposed, that a module of the group of modules 3 comprises a chromatography medium 23 and/or a filter medium and/or a pump 24 and/or a detector 25 for generating the detector signal, and/or a bioreactor, and/or a freeze drier, and/or a scale.

It is preferred, that an operation module 6 of the number of operation modules comprises the chromatography medium 23 and/or the filter medium, and/or, that a support module 9 of the number of support modules comprises the pump 24 and/or the detector 25 for generating the detector signal and/or the bioreactor and/or the freeze drier and/or the scale.

It is more preferred, that the chromatography medium 23 and/or the filter medium is comprised by the operation modules 6 of the number of operation modules only, and/or, that the pump 24 and/or the detector 25 for generating the detector signal and/or the bioreactor and/or the freeze drier and/or the scale is comprised by a support module 9 of the number of support modules and/or the start module 4 and/or the end module 5 only.

The chromatography medium 23 may be a cation exchange chromatography medium 23, an anion exchange chromatography medium 23, a hydrophobic chromatography medium 23, a mixed-mode chromatography medium 23 or an affinity chromatography medium 23. The affinity chromatography medium 23 may comprise an immunoglobulin binding protein such as protein A. The filter medium may be a depth filter medium, a sterile filter medium or a virus filtration medium.

In some embodiments, one unit operation is assigned to one operation module 6 of the number of operation modules. Preferably, the unit operation is a chromatography step, a filtration step or a virus inactivation step. For example, and as shown in Fig. 3a), the support module 9 may comprise a pump 24 for transporting the process liquid to the operation module 6 via the physical interfaces 7 of the group of predefined physical interfaces of the support module 9 and the operation module 6. The operation module 6 may, as also shown in Fig. 3a), comprise a chromatography medium 23 for performing a chromatography step. Subsequently, the process liquid may then be transferred from the operation module 6 to the end module 5 via the physical interfaces 7 of the operation module 6 and the end module 5. As shown in Fig. 3a) the process liquid may then be analyzed by a detector comprised by the end module 5.

In some embodiments, the chromatography step is performed in bind and elute mode or in flow through mode. The filtration step may be performed in normal flow filtration (NFF) or tangential flow filtration (TFF) mode. In some embodiments, the filtration step is an ultrafiltration step or a diafiltration step.

The virus inactivation step may comprise an adjustment of the pH of the process liquid to a virus inactivating pH value and/or an addition of a virus inactivating agent to the process liquid to obtain a virus inactivating condition in the process liquid. The virus inactivating pH value may be a pH value or the process liquid between 3.0 and 5.0, preferably a pH value above 3.1 and below 3.6. The virus inactivating agent may be a solvent and/or a detergent. Furthermore, the virus inactivation step may comprise the step of incubating of the process liquid at the virus inactivating condition for a predefined time.

According to one embodiment it is proposed, that each module of the group of modules 3 comprises an, in particular lateral, arrangement surface 26 for assigning the relative position of the modules of the group of modules 3 to each other and that the physical interfaces 7 of the group of predefined physical interfaces, in particular the group of electric connectors and/or the group of fluidic connectors, are arranged within the arrangement surfaces 26 only. Such embodiments allow grouping electric connectors 10 of the group of electric connectors and/or grouping fluidic connectors 11 of the group of fluidic connectors of a module of the group of modules 3 to form a plug connector that comprises all connectors required for one or multiple functions performed by the respective module. Furthermore, such an arrangement surface 26 allows allocating the grouped connectors to a predefined location.

In addition, and as can be seen in Fig. 3a), the physical interfaces 7 between adjacent modules may be arranged on each module in a way that a seamless connection of the adjacent modules in a connected state of the modules is achieved. Such seamless connection of modules may simplify the sterilization and/or cleaning of the bioprocessing system 1 which may facilitate compliance with GMP-criteria.

As shown in Fig. 3a) an operation module 6 of the number of operation modules and/or a support module 9 of the number of support modules may comprise multiple arrangement surfaces 26, each comprising a physical interface 7.

By arranging an arrangement surface 26 on opposing sides of each module between the start module 4 and the end module 5, a linear configuration of the bioprocessing system 1, and thereby a linear extension of the footprint 8 can be achieved (Figures 1 and 3). However, by arranging an arrangement surface 26 on two sides of a module of the group of modules 3, that are angled towards each other, an angled or round-shaped configuration of the bioprocessing system 1, and thereby an angled or round extension of the footprint 8 can be achieved (Fig. 2). This allows a bioprocessing system 1 to be provided which may optimally be adapted to the spatial conditions of a room, such as a laboratory or a production room. Furthermore, this may also allow a bioprocessing system 1 to be adapted to the spatial conditions of a transportation route along which the bioprocessing system 1 needs to be transported, for example for transporting the bioprocessing system 1 from a non-sterile production area to a sterile production area and vice versa.

As shown in Figures 1 to 3, a module of the group of modules 3 may comprise a housing 27 for housing at least one sub-module 28. The sub-module 28 is configured to perform an operation, in particular a unit operation of the number of unit operations, of the connected bioprocess 2. The sub-module 28 may comprise a component required for performing the operation, in particular a unit operation of the number of unit operations. For example, the sub-module 28 may comprise a pump 24 and/or a chromatography medium 23 and/or a filter medium and/or a detector 25 for generating the detector signal, and/or a bioreactor, and/or a freeze drier, and/or a scale. In this regard, it is preferred that the sub-module 28 can be inserted, in particular be slid, into the housing 27.

In preferred embodiments, the physical interfaces 7 of the group of predefined physical interfaces are arranged on the housing 27. In other embodiments the housing 27 may comprise a group of housing interfaces (not shown) and the sub-module 28 may comprise a group of sub-module 28 interfaces (not shown) for fluidically and/or electrically interconnecting the sub-module 28 with the housing 27, in particular with a physical interface 7 of the group of predefined physical interfaces, and/or a fluid source interface 18 of the group of fluid source interfaces and/or an electric source interface 20 of the group of fluid source interfaces comprised the housing 27. Preferably, all housing interfaces of the group of housing interfaces are arranged in the interior of the housing 27 and all sub-module 28 interfaces of the group of sub-module 28 interfaces are arranged on the exterior if the sub-module 28.

According to one embodiment, it is proposed that the operation modules 6 of the number of operation modules are spatially arranged between the start module 4 and the end module 5.

In some embodiments, all modules have a common, in particular planar, surface as shown in Fig. 1. Preferably, the common surface 29 is formed by a top surface of the housing 27 of at least two, in particular all, modules of the group of modules 3. This allows, as illustrated in Fig. 1, to place further components on the top surface. For example, a monitor 30 and/or a fluid storage compartment 31 and/or the liquid handling station 21 may be placed on the top surface. However, in other embodiments (not shown), a further component may be attached to a side surface of a module of the group of modules 3, in particular to a surface of a module of the group of modules 3, which is typically directed towards a user in an operational state of the bioprocessing system 1 (front surface).

Another teaching, which is of equal importance, relates to a method for setting up a proposed bioprocessing system 1, wherein the method comprises configuring the bioprocessing system 1 for performing a first connected bioprocess 2 by interconnecting a start module 4 and at least two operation modules 6 of the number of operation modules via the physical interfaces 7 of the group of predefined physical interfaces and reconfiguring the bioprocessing system 1 for performing a second connected bioprocess 2 by adding or removing one of the operation modules 6.

Another teaching which is of equal importance relates to a process unit 32 for controlling a proposed bioprocess system 1.

The process unit 32 may be configured to initiate the support functions of the start module 4 and/or the end module 5 and/or a support module 9 of the number of support modules. The support functions may, for example, comprise a controlling support function, and/or a process liquid-related support function, and/or an assisting support function.

Therefore, the bioprocessing system 1 may further comprise a computer program comprising commands, which, when the program is executed by the process unit 32, cause the process unit 32 to carry out the initiation of the support functions. Preferably, the process unit 32 is comprised by the start and/or end module 5. Therefore, the computer program may be configured to orchestrate all electrical signals 13. The computer program may allow a user identification, an automated module recognition and/or the control of a user interface in which control information for controlling the bioprocessing system 1 can be inputted, in particular by a user.

## Claims

1. Bioprocessing system for performing a connected bioprocess (2) of a process liquid, wherein the bioprocessing system (1) comprises a group of modules (3), wherein the group of modules (3) comprises a start module (4) and/or an end module (5) for performing support functions during the connected bioprocess (2), wherein the group of modules (3) comprises a flexible number of operation modules each being functionally arranged behind the start module (4) and/or in front of the end module (5), wherein the bioprocessing system (1) is configured to perform a flexible number of unit operations by the flexible number of operation modules, wherein the start module (4) and/or the end module (5) is or are configured to perform the support functions for each operation module (6), wherein each module of the group of modules (3) comprises a group of predefined physical interfaces for fluidically and/or electrically interconnecting the modules of the group of modules (3) with each other, wherein the bioprocessing system (1) has a footprint (8) that is defined by the interaction of the physical interfaces (7) of the group of predefined physical interfaces.

2. Bioprocessing system according to claim 1, **characterized in that** the group of modules (3) comprises a flexible number of support modules for performing support functions during the connected bioprocess (2), which are functionally arranged behind the start module (4) and/or in front of the end module (5).

3. Bioprocessing system according to claim 1 or 2, **characterized in that** the group of predefined physical interfaces comprises a group of electric connectors, in particular detachable, electrical plug-socket connectors, for electrically interconnecting the modules of the group of modules (3) with each other, preferably, that all electric connectors (10) of the group of electric connectors are of the same type,
and/or,
that the group of predefined physical interfaces comprises a group of fluidic connectors, in particular detachable, fluidic plug-socket connectors, for fluidically interconnecting the modules of the group of modules (3) with each other, preferably, that all fluidic connectors (11) of the group of fluid connectors are of the same type.

4. Bioprocessing system according to any one of the preceding claims, **characterized in that** the number of operation modules comprises a coupling operation module (12), and that the coupling operation module (12) is configured to receive an electric signal (13) and/or a fluid from a transmitting module of the group of modules (3) and to transmit the electric signal (13) and/or the fluid to a receiving module of the group of modules (3) via the physical interfaces (7) of the group of predefined physical interfaces of the transmitting module, the coupling operation module (12) and the receiving module,
and/or,
that the number of support modules comprises a coupling support module (9), and that the coupling support module (9) is configured to receive an electric signal (13) and/or a fluid from the transmitting module of the group of modules (3) and to transmit the electric signal (13) and/or the fluid to the receiving module of the group of modules (3) via the physical interfaces (7) of the group of predefined physical interfaces of the transmitting module, the coupling support module (9) and the receiving module.

5. Bioprocessing system according to any one of the preceding claims, **characterized in that** the electric signal (13) is received from the start module (4) and/or the end module (5) and/or an operation module (6) and/or a support module (9) as an electric source module (14), and/or, that the fluid is received from the start module (4) and/or the end module (5) and/or an operation module (6) and/or a support module (9) as a fluid source module (15), preferably, that the fluid received from the fluid source module (15), in particular from an operation module (6) as fluid source module (15), is introduced into the fluid source module (15) from an external feedstream (16), preferably, that the external feedstream (16) is the process liquid derived from an upstream process.

6. Bioprocessing system according to claim 4 or 5, **characterized in that** the electric signal (13) is a control signal for controlling a module of the group of modules (3), preferably, for controlling a unit operation of an operation module (6) of the group of operation modules (6), and/or,
that the signal is a detector signal representing a bioprocess parameter of the process liquid.

7. Bioprocessing system according to one of the preceding claims, **characterized in that** a module of the group of modules (3) comprises a chromatography medium (23) and/or a filter medium and/or a pump (24) and/or a detector (25) for generating the detector signal, and/or a bioreactor, and/or a freeze drier, and/or a scale, preferably,
that an operation module (6) of the number of operation modules comprises the chromatography medium (23) and/or the filter medium, and/or, that a support module (9) of the number of support modules and/or the start module (4) and/or the end module (5) comprises the pump (24) and/or the detector (25) for generating the detector signal and/or the bioreactor and/or the freeze drier and/or the scale.

8. Bioprocessing system according to one of the preceding claims, **characterized in that** one unit operation is assigned to one operation module (6) of the number of operation modules, preferably, that the unit operation is, a chromatography step, a filtration step or a virus inactivation step.

9. Bioprocessing system according to one of the preceding claims, **characterized in that** each module of the group of modules (3) comprises an, in particular lateral, arrangement surface (26) for assigning the relative position of the modules of the group of modules (3) to each other, and that the physical interfaces (7) of the group of predefined physical interfaces, in particular the group of electric connectors and/or the group of fluidic connectors, are arranged within the arrangement surfaces (26) only.

10. Bioprocessing system according to one of the preceding claims, **characterized in that** a module of the group of modules (3) comprises a housing (27) for housing at least one sub-module (28), wherein the sub-module (28) is configured to perform an operation, in particular a unit operation of the number of unit operations, of the connected bioprocess (2), preferably, that the sub-module (28) can be inserted, in particular be slid, into the housing (27).

11. Bioprocessing system according to claim 10, **characterized in that** the physical interfaces (7) of the group of predefined physical interfaces are arranged on the housing (27).

12. Bioprocessing system according to one of the preceding claims, **characterized in that** the operation modules (6) of the number of operation modules are spatially arranged between the start module (4) and the end module (5).

13. Bioprocessing system according to one of the preceding claims, **characterized in that** all modules have a common, in particular planar, surface, preferably that the common surface (29) is formed by a top surface of the housing (27) of at least two, in particular all, modules of the group of modules (3).

14. Method for setting up a bioprocessing system (1) according to one of the preceding claims, wherein the method comprises configuring the bioprocessing system (1) for performing a first connected bioprocess (2) by interconnecting a start module (4) and at least two operation modules (6) of the number of operation modules via the physical interfaces (7) of the group of predefined physical interfaces and reconfiguring the bioprocessing system (1) for performing a second connected bioprocess (2) by adding or removing one of the operation modules (6).

15. Process unit for controlling a bioprocess system (1) according to claims 1 to 13.
